# EUROPEAN PATENT SPECIFICATION

(11) **EP 1 483 011 B1**
(45) Date of publication and mention of the grant of the patent: **20.01.2010**
(21) Application number: 03739763.5
(22) Date of filing: 11.02.2003
(51) Int. Cl.: A61M 35/00

(54) **PRE-MOISTENED APPLICATORS FOR CHEMICAL REACTANT DELIVERY**
VORBEFEUCHTETE APPLIKATOREN FÜR DIE ABGABE VON CHEMISCHEN REAKTIONSPARTNERN
APPLICATEURS PRE-HUMIDIFIES POUR LA FOURNITURE DE REACTIF CHIMIQUE

(30) Priority: 11.02.2002 US 73717
(43) Date of publication of application: 08.12.2004
(73) Proprietor: Illinois Tool Works Inc., Glenview, IL 60026 (US)
(72) Inventor: Willard, Dean M., New York, NY 10017 (US); Feist, Mark W., Colchester, CT 06415 (US); Hass, Hans E., Stow, OH 44224 (US); Snyder, Marcia, Stow, OH 44224 (US); Zook, Jonathan D., Santa Clarita, CA 91351 (US)
(74) Representative: Naismith, Robert Stewart
(86) International application number: PCT/US2003/004208
(87) International publication number: WO 2003/068308

(56) References cited:
- WO-A-01/27239
- WO-A-97/04831
- US-A- 4 696 393
- US-A- 4 696 393
- US-B1- 6 283 933
- US-B1- 6 283 933
- US-B2- 6 415 808

## Description

The present invention generally relates to chemical reactant application, and more particularly, to pre-moistened applicators for such a reactant.

Many polymerization reactions progress under anaerobic conditions. The desire to induce polymerization under field conditions or absent equipment necessary to control the atmosphere during polymerization requires that polymerization components be pre-packaged to inhibit degradation associated with oxygen or humidity exposure.

It is common that single-use polymerization adhesives that cure best under anaerobic conditions are packaged to assure that the air-sensitive polymerization catalyst is maintained during storage. Such polymerization catalysts have traditionally been provided in swab ampules that include an interior, breakable, sealed glass capsule containing the catalyst in flowable liquid form. An outer protective sheath or vial is formed from plastic sheeting such as polyethylene, polyvinyl chloride, or other conventional polymeric materials. This outer protective sheath is sealed at one end and terminates at the other end in a spongeous, liquid permeable piece. In operation, a substrate is pre-cleaned and the glass capsule within the ampule is broken thereby releasing catalyst containing liquid to feed by gravity into the spongeous applicator piece while the fragmented glass capsule is ideally retained within the polymeric protective sheath. Conventional ampules of this type are typically many times more expensive to prepare than the cost of the polymerization catalyst contained therein. Additionally, a safety hazard exists in the event that the outer polymeric sheath is punctured during the shattering of a glass vial with a user's fingers. A further limitation of conventional ampules is the need to orient the ampule above the substrate to maintain liquid flow. In light of the expense, hazard, and application orientation associated with delivery of anaerobic polymerization catalysts in such ampules, there exists a need for a chemical reactant applicator that retains the function of the reactant during storage and overcomes the limitations of conventional ampules.

PCT Patent Application Publication No. WO-A-97/04831 discloses a topical hyperbaric bandage comprising a gas diffusion resistant flexible and/or resistant sheet material including an adhesive layer adapted to be affixed to the skin, said adhesive layer adapted to surround a treatment area, at least one release layer disposed over said adhesive layer, and means for the supply of therapeutic gas to the treatment area, the said device being adapted to retain a single charge of therapeutic gas over a treatment period; characterized in that said bandage comprises a reservoir for a source of therapeutic gas in liquid form, and in that gas from the reservoir is initiable after the bandage has been secured to the skin.

### Summary of the Invention

An article for chemical reactant delivery includes a liquid chemical reactant under delivery conditions or a solvated chemical reactant that is premoistened into an applicator. A chemical reactant impervious package has a pouch adapted to enclose the applicator. The applicator wicks the chemical reactant to the substrate contact surface through capillary action and is in the form of a wipe, a prep pad, a swab stick or a sponge. A process for applying an adhesive to a substrate involves exposing a capillary action fed applicator premoistened with a solvated or liquid chemical reactant and contacting the applicator with a first substrate independent of user contact with the applicator. A film of the chemical reactant is then applied to the substrate and a second substrate is brought into contact with the first substrate with a curable material and the chemical reactant therebetween. A commercial package including an article for chemical reactant delivery includes a solvated or liquid under delivery conditions chemical reactant pre-moistened into an applicator that is stored within a chemical reactant impervious package having a pouch adapted to enclose the applicator, along with instructions for the use thereof as a premoistened applicator for the chemical reactant. The use of a selectively peelable or tearable package for applying a chemical reactant pre-moistened into an applicator is also disclosed.

### Brief Description of the Drawings

Figs. 1A-C are planar, cross-sectional, and perspective views, respectively, of an inventive embodiment; and
Figs. 2A-C are planar, cross-sectional, and perspective views, respectively, of another inventive embodiment.

### Description of the Preferred Embodiments

The present invention overcomes the limitations of conventional ampules by packaging a chemical reactant as a pre-moistened wipe, prep pad, swab or stick that is stored within a package formed from metal foil having an inner coating or layer of catalyst and catalyst solvent compatible thermoplastic material. Alternatively, the pre-moistened applicator is within a package formed of a puncture- or tear-resistant thickness of a suitable catalyst and catalyst solvent compatible thermoplastic material. The present invention utilizes capillary action within an applicator to deliver a chemical reactant, in contrast to gravity fed applicators common to the art.

As used herein, a pre-moistened applicator is defined as a wipe, a preparation pad, a swab, a swab stick and a sponge wetted with a liquid chemical reactant or a solvated chemical reactant.

As used herein, a chemical reactant is defined as a reaction catalyst, a primer, an activator, an adhesion promoter and a polymerization monomer.

In the attachment of non-siliceous materials to a glass substrate the present invention finds a particular utility. The attachment of a rearview mirror mount to a vehicle windshield is an exemplary utility of the present invention. An inventive package is torn or peeled to expose an applicator pre-moistened with the anaerobic cure adhesive catalyst. The applicator contacts the glass substrate to leave a thin film of catalyst material in a desired bonding region of the substrate. Optionally, any solvent accompanying the catalyst onto the substrate is given sufficient time to volatilize. The curable adhesive is then applied of the catalyst and the metal (or other material) mount substrate is brought into contact with the glass substrate such that the catalyst indicated anaerobically curing adhesive is therebetween for a time sufficient to allow cure to occur.

An additional advantage of the present invention over the conventional swab ampule is the ability to apply a chemical reactant with greater uniformity in substrate geometrics where a gravity fed ampule swab is not in fluid contact with the catalyst reservoir. The solvated or liquid chemical reactant is applied to a pre-moistened applicator according to the present invention by soaking the applicator in a solution, spraying the solution onto the applicator, or by an injection technique into the article during manufacture. The identity of the solvent is largely dictated by the solubility characteristics of the chemical reactant and compatibility of that solvent with the other chemical reactants. Solvents operative herein illustratively include: water, C₂-C₂₀ linear or branched alkanes; ethers; esters; alcohols; ketones; aldehydes; acids; C₆-C₁₀ aromatics and substituted aromatics; furans; and chlorinated, brominated, and fluorinated forms thereof; plasticizers; oils, such as dioctyl phthalate (DOP); and liquid resins such as triethylene glycol mono-methacrylate (triEGMA) and polyethylene glycol mono-methacrylate (PEGMA).

The chemical reactant illustratively includes the following classes of materials: main group and lanthanide series organometallics, coordination complexes of main group and lanthanide series metal salts and more specifically organo-tin compounds and organo-copper compounds, and copper acetyl acetonate or aldehyde amine condensates, which are known to the art to be active as anaerobic polymerization catalysts and/or primers. It is appreciated that a chemical reactant that is a liquid under application conditions is optionally not solvated or otherwise diluted within the present invention.

Contrary to prior art, pre-moistened applicators which contained personal care astringents, biocides or cleaning solutions, an inventive solvated chemical reactant is prone to degradation by skin contact or act as a skin irritant. As a result, care is taken upon opening the package to avoid skin contact with the pre-moistened applicator contained therein. Skin contact is avoided either through use of applicator solvent- and catalyst-impervious gloves or cots, forceps, tweezers or preferably, a package peelable to expose the pre-moistened applicator while shielding the same from user contact.

While the present invention is described with a catalyst component of a multi-component chemical reaction being in a pre-moistened applicator package, it is appreciated that the present invention is also operative to contain a variety of multiple function components involved in a chemical reaction. Exemplary multiple function pre-moistened applicators include in combination a substrate cleaner-primer, a substrate cleaner-catalyst, and a substrate cleaner-adhesion promoter. It is further appreciated that substrate cleaning may be completed by a chemical reactant solvent alone or in combination with a surfactant, an acid or a base.

Absorbent materials in packaging systems operative herein include those detailed in U.S. Patent Nos. 3,542,634; 3,057,467; and 4,696,393, Preferably, the package according to the present invention retains the package in the contained pre-moistened applicator attached thereto as prior U.S. Patent No. 4,696,393. In the context of the present invention, the applicator pre-moistened with a solvated chemical reactant is exposed by flaring the package flaps to expose the pre-moistened applicator therein. By holding onto the base of the package, the pre-moistened applicator contents are spread onto a substrate and after use, the package opening flaps are again brought into contact, thereby containing the spent applicator therein, all without skin contact with the applicator.

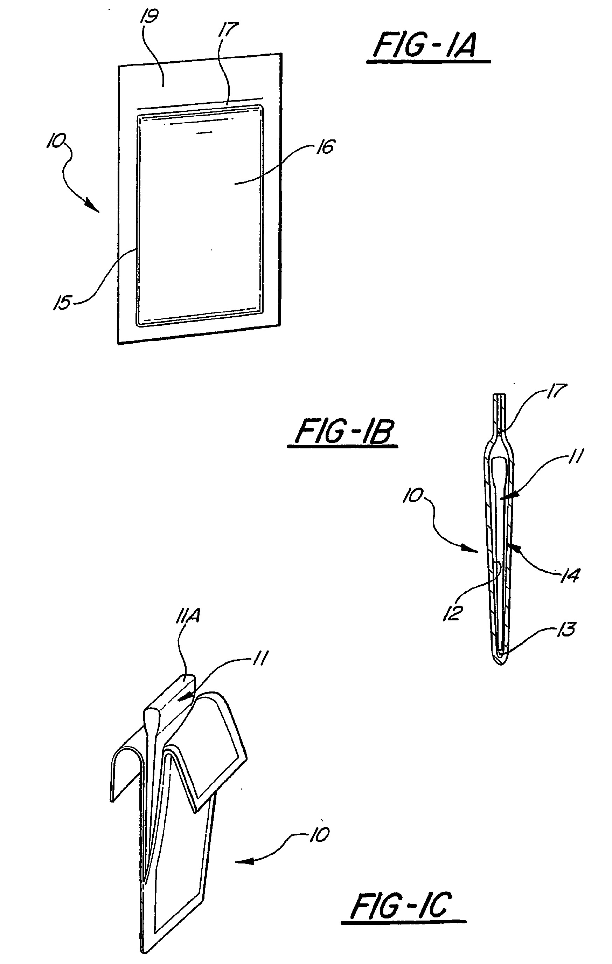

Referring now to the drawings, where like referenced numerals denote similar components among several figures, in Figs. 1A-1C, an inventive pre-moistened applicator article is shown generally at 10. The article 10 includes a pre-moistened applicator pad 11 optionally attached to the package inner surface 12. Optionally, the applicator tip 11A that is exposed upon pulling back on package flaps, is of a greater thickness than the remainder of the applicator pad in order to wick additional catalyst onto the portion the applicator contacting a substrate. The attachment 13 between the applicator 11 and the inner surface 12 of package 14 illustratively includes contact adhesive, solvent weld, thermal fusion, thermoset adhesive. An intendedly permanent seal 15 proximal to the edges of the package 14 extends around a portion of the package periphery to define a pouch 16 having an opening 17. The opening 17 is selectively sealed for storage with a breakable seal material 18. The breakable seal 18 is illustratively an adhesive seal, a heat seal, pressure seal or combination thereof that is compatible with the solvent and catalyst impregnated in the applicator 11, as well as the package materials. Preferably, a flap 19 extends beyond the breakable seal 17 to facilitate opening of the seal 17 to expose the pre-moistened applicator 11.

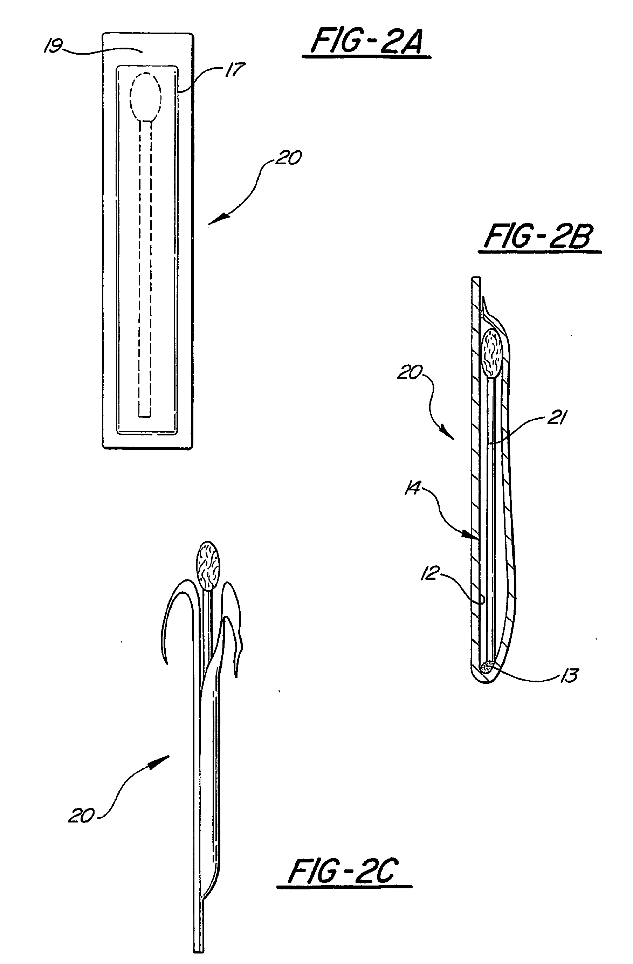

With reference to Figs. 2A-2C, a pre-moistened applicator swab stick 21 is encased within an article shown generally at 20.

The following examples are intended to give operative embodiments and comparatives of the present invention. The compositions and properties detailed herein are only intended to be exemplary and not limiting as to the scope of the invention detailed in the appended claims.

### Example 1. Comparison Between Conventional Ampule and Inventive Applicator Adhesive.

### Test Methods and Materials

Commercial alcohol prep pads were obtained, the cotton pads removed from the package and dried. The pads were impregnated with various primers and the surfaces of steel lap shears that were pre-cleaned with isopropanol were wiped with the primer pads in a uniform motion. The lap shears were bonded with an anaerobic adhesive and allowed to dry for 24 hours at ambient temperature. The force to pull the laps apart was determined using an Instron Tensile Tester in accordance with ASTM D1002, which is incorporated herein by reference.

### Components/Raw Materials

Cotton prep-pads: 1¼" x 2⅛", impregnated with ∼0.40 grams primer solution Conventional primer ampules
Adhesive A: Control; Permatex part #81844 - 1 drop per lap shear set
Primer A: Control; Permatex part #81844 activator
Primer B: Copper naphthenate in isopropanol
Primer C: Copper octoate in isopropanol
Primer D: Copper octoate in heptane

**Table 1**

| **Primer Applied by Conventional Ampule vs Inventive Applicator Primer Wiped On** | | |
|---|---|---|
| | **Primer Application** | **Tensile Strength (psi)** |
| Primer A | ampule | 2,552 +/- 141 |
| Primer A | ampule | 2,543 +/- 259 |
| Primer A | inventive applicator | 2,310 +/- 162 |
| Primer A | inventive applicator | 2,605 +/- 334 |
| Primer B | ampule | 2,679 +/- 398 |
| Primer B | inventive applicator | 3,035 +/- 155 |
| Primer B | inventive applicator "dry pad" (pad dried 2.5 minutes then applied) | 2,672 +/- 341 |
| Primer B | inventive applicator | 2,727 +/- 184 |
| Primer B | inventive applicator "aged wipe" (pad aged @ 50°C 1 month) | 2,327 +/- 361 |
| Primer B | inventive applicator | 2,604 +/- 226 |

| | | |
|---|---|---|
| Results reported are averages of 3-5 laps per set. Primers used in conjunction with Adhesive A in all cases. | | |

**Table 2**

| **Baseline Tensile Strengths for Conventional Ampule Applied Primers on Clean Lap Shears** | |
|---|---|
| **Primer Type Comparison - Conventional Ampule Applied Primer Used with Adhesive A** | **Tensile Strength (psi)** |
| Primer A - Trial 1 | 2,815 +/- 102 |
| Primer A - Trial 2 | 2,619 +/- 338 |
| Primer B - Trial 1 | 2,500 +/- 194 |
| Primer B - Trial 2 | 2,798 +/- 371 |
| Primer C | 2,430 +/- 271 |
| Primer D | 2,628 +/- 334 |

### Example 2. Inventive Applicator as Cleaner and Primer.

The same test method was used per Example 1; however, instead of pre-cleaning a lap shear, it was dipped into an isopropanol contaminant solution. A lap was allowed to dry for 1 hour and the excess material "dabbed" off with a dry paper towel. The activator-impregnated inventive wipe was then used as the "cleaning towel", which also left a primer film on the surface of Primer A where Adhesive A was used in all cases. Laps were assembled and tested as above.

**Table 3**

| **Tensile Strengths for Inventive Applicator Applied Primers on Contaminated Lap Shears** | |
|---|---|
| **Contaminant** | **Tensile Strength (psi)** |
| 5% 1000 centistoke silicone oil | 2,401 +/- 126 |
| 5% motor oil | 1,718 +/- 287 |
| 5% dioctyl phthalate | 2,817 +/- 152 |
| No contaminant | 3,230 +/- 115 |

The foregoing description is illustrative of particular embodiments of the invention, but is not meant to be a limitation upon the practice thereof. The following claims, including all equivalents thereof, are intended to define the scope of the invention.

## Claims

1. An article (10) for chemical reactant delivery comprising:
an anaerobic cure adhesive catalyst chemical reactant including one of the main group and lanthanide series organometallics, coordination complexes of main group and lanthanide series metal salts, and more specifically organo-tin compounds or organo-copper compounds, and copper acetyl acetonate or aldehyde amine condensates that satisfies at least one condition of being solvated or liquid under delivery conditions;
an applicator (11) pre-moistened with said chemical reactant;
a chemical reactant impervious package (14) having a pouch (16) adapted to enclose said applicator, said package peelable to expose the pre-moistened applicator while shielding the same from user contact.

2. The article of claim 1 wherein said package has a pouch defined in part by a temporary seal (18).

3. The article of claim 1 wherein said pre-moistened applicator is attached to said package.

4. The article of claim 1 wherein said applicator is selected from a group consisting of a wipe, prep pad, a swab stick, and a sponge.

5. The article of claim 2 wherein said package has a peelable flap (19) proximal to the said temporary seal.

6. A process for applying an adhesive comprising the steps of:
exposing a capillary action fed applicator (11) pre-moistened with a solvated or liquid chemical reactant comprising an anaerobic cure polymerization monomer and an anaerobic cure adhesive catalyst including one of main group and lanthanide series organometallics, coordination complexes of main group and lanthanide series metal salts and more specifically organo-tin compounds and organo-copper compounds, and copper acetyl acetonate or aldehyde amine condensates;
contacting said applicator with a first substrate independent of a user contact of said applicator;
applying a film of said chemical reactant to said first substrate; and
bringing a second substrate into contact with the first substrate with a curable material and said chemical reactant therebetween to induce anaerobic cure.

7. The process of claim 6 wherein said applicator is oriented below the first substrate while applying said film of said chemical reactant.

8. A commercial package comprising an article (10) according to claim 1 together with instructions for the use thereof as a pre-moistened applicator for a chemical reactant.

9. The use of a selectively peelable or tearable package for applying a chemical reactant pre-moistened in an applicator (11).

10. The use of claim 9 where said chemical reactant is delivered independent of user contract therewith.

11. An adhesive bond obtainable by the process as claimed in claim 6.

## Patentansprüche

1. Artikel (10) für die Abgabe von chemischen Reaktionspartnern, umfassend:
einen chemischen Reaktionspartner eines durch anaerobe Härtung haftenden Katalysators, welcher eines aus den Organometallen der Hauptgruppe und
der Lanthanoid-Reihe, Koordinationskomplexen von Metallsalzen der Hauptgruppe und der Lanthanoid-Reihe und insbesondere Organo-Zinn-Verbindungen oder Organo-Kupfer-Verbindungen und
Kupferacetylacetonat- oder Aldehydaminkondensaten einschließt, welcher mindestens einer Bedingung genügt, und zwar unter Abgabebedingungen solvatisiert oder flüssig zu sein;
einen Applikator (11), welcher mit dem chemischen Reaktanten vorbefeuchtet wurde;
eine für chemische Reaktionspartner undurchlässige Verpackung (14) mit einem Säckchen (16), das so angepasst ist, um den Applikator zu umschließen, wobei die Verpackung ablösbar ist, um den vorbefeuchteten Applikator zu exponieren, während selbiger gegenüber einem Kontakt durch den Benutzer abgeschirmt wird.

2. Artikel gemäß Anspruch 1, wobei die Verpackung ein zum Teil durch eine temporäre Versiegelung (18) definiertes Säckchen aufweist.

3. Artikel gemäß Anspruch 1, wobei der vorbefeuchtete Applikator an der Verpackung angebracht ist.

4. Artikel gemäß Anspruch 1, wobei der Applikator aus der Gruppe gewählt ist, die aus einem Wischer, Tupfer, einem Wattestäbchen und einem Schwamm besteht.

5. Artikel gemäß Anspruch 2, wobei die Verpackung eine ablösbare Klappe (19) proximal zu der temporären Versiegelung aufweist.

6. Verfahren zum Aufbringen eines Klebstoffs, umfassend die folgenden Schritte:
Exponieren eines durch Kapillarwirkung gespeisten Applikators (11), welcher mit einem solvatisierten oder flüssigen chemischen Reaktionspartner vorbefeuchtet wurde, umfassend ein Polymerisationsmonomer mit anaerober Härtung und
einen durch anaerobe Härtung haftenden Katalysator, welcher eines aus den Organometallen der Hauptgruppe und der Lanthanoid-Reihe, Koordinationskomplexen von Metallsalzen der Hauptgruppe und der Lanthanoid-Reihe und
insbesondere Organo-Zinn-Verbindungen oder Organo-Kupfer-Verbindungen und Kupferacetylacetonat- oder Aldehydaminkondensaten einschließt;
Kontaktieren des Applikators mit einem ersten Substrat unabhängig von einem Benutzerkontakt des Applikators;
Aufbringen eines Films des chemischen Reaktionspartners auf das erste Substrat; und
In-Kontakt-Bringen eines zweiten Substrats mit dem ersten Substrat mit einem härtbaren Material und
dem chemischen Reaktionspartner dazwischen zur Herbeiführung einer anaeroben Härtung.

7. Verfahren gemäß Anspruch 6, wobei der Applikator unterhalb des ersten Substrats ausgerichtet wird, während der Film des chemischen Reaktionspartners aufgebracht wird.

8. Kommerzielle Verpackung, umfassend einen Artikel (10) gemäß Anspruch 1, zusammen mit Anleitungen für dessen Verwendung als ein vorbefeuchteter Applikator für einen chemischen Reaktionspartner.

9. Verwendung einer selektiv ablösbaren oder zerreißbaren Verpackung zum Aufbringen eines chemischen Reaktionspartners, vorbefeuchtet in einem Applikator (11).

10. Verwendung gemäß Anspruch 9, wobei der chemische Reaktionspartner unabhängig von einem Benutzerkontakt damit abgegeben wird.

11. Klebeverbindung, die durch das Verfahren gemäß Anspruch 6 erhältlich ist.

## Revendications

1. Article (10) pour la fourniture de réactif chimique, comprenant :
un réactif chimique catalyseur pour un adhésif à durcissement anaérobie comprenant l'un des composés organométalliques du groupe principal et de la classe des lanthanides, des complexes de coordination de sels de métaux du groupe principal et de la classe des lanthanides, et plus précisément des composés organiques d'étain ou des composés organiques de cuivre, et des condensats d'acétylacétonate de cuivre ou d'aldéhyde-amine, qui satisfait à au moins l'une des conditions d'être solvaté ou d'être liquide dans les conditions de fourniture ;
un applicateur (11) pré-humidifié avec ledit réactif chimique ;
un emballage étanche au réactif chimique (14), comportant une poche (16) adaptée pour abriter ledit applicateur, ledit emballage étant pelable pour exposer l'applicateur pré-humidifié tout en empêchant celui-ci d'entrer en contact avec un utilisateur.

2. Article selon la revendication 1, dans lequel ledit emballage comporte une poche définie en partie par un joint d'étanchéité temporaire (18).

3. Article selon la revendication 1, dans lequel ledit applicateur pré-humidifié est fixé audit emballage.

4. Article selon la revendication 1, dans lequel ledit applicateur est sélectionné dans un groupe constitué d'une lingette, d'un tampon de préparation, d'un bâtonnet écouvillon et d'une éponge.

5. Article selon la revendication 2, dans lequel ledit emballage comporte un volet pelable (19) proximal audit joint d'étanchéité temporaire.

6. Procédé d'application d'un adhésif, comprenant les étapes qui consistent à :
exposer un applicateur alimenté par capillarité (11) pré-humidifié avec un réactif chimique solvaté ou liquide comprenant un catalyseur pour un monomère polymérisable par durcissement anaérobie et un adhésif à durcissement anaérobie comprenant l'un des composés organométalliques du groupe principal et de la classe des lanthanides, des complexes de coordination de sels de métaux du groupe principal et de la classe des lanthanides, et plus précisément des composés organiques d'étain et des composés organiques de cuivre, et des condensats d'acétylacétonate de cuivre ou d'aldéhyde-amine ;
mettre en contact ledit applicateur avec un premier substrat sans nécessiter de contact entre un utilisateur et ledit applicateur ;
appliquer un film dudit réactif chimique sur ledit premier substrat ; et
mettre en contact un deuxième substrat avec le premier substrat, un matériau durcissable et ledit réactif chimique étant présents entre ceux-ci pour induire un durcissement anaérobie.

7. Procédé selon la revendication 6, dans lequel ledit applicateur est orienté sous le premier substrat durant l'application dudit film dudit réactif chimique.

8. Emballage commercial comprenant un article (10) selon la revendication 1 conjointement avec des instructions pour l'utilisation de celui-ci comme applicateur pré-humidifié pour un réactif chimique.

9. Utilisation d'un emballage pelable ou déchirable sélectivement pour l'application d'un réactif chimique avec lequel un applicateur a été pré-humidifié (11).

10. Utilisation selon la revendication 9, dans laquelle ledit réactif chimique est fourni sans nécessiter de contact entre un utilisateur et celui-ci.

11. Liaison adhésive pouvant être obtenue par le procédé selon la revendication 6.
